# FASCICULE DE BREVET EUROPEEN

(11) **EP 2 090 574 B1**
(45) Date de publication et mention de la délivrance du brevet: **21.09.2011**
(21) Numéro de dépôt: 09150741.8
(22) Date de dépôt: 16.01.2009
(51) Int. Cl.: C07D 317/54

(54) **Procédé de préparation de particules de stiripentol ayant une granulométrie définie**
Herstellungsverfahren von Stiripentolpartikeln, die eine definierte Granulometrie haben
Method for preparing stiripentol particles with defined particle size

(30) Priorité: 23.01.2008 FR 0850413
(43) Date de publication de la demande: 19.08.2009
(73) Titulaire: Biocodex, 94250 Gentilly (FR)
(72) Inventeur: Hublot, Bernard, 60200, COMPIEGNE (FR); Berthon-Cedille, Laurence, 60490, RICQUEBOURG (FR); Leguern, Marie-Emmanuelle, 60200, COMPIEGNE (FR); Renaud, Gilles, 92400, COURBEVOIE (FR)
(74) Mandataire: Vial, Lionel

(56) Documents cités:
- FR-A- 2 173 691

## Description

La présente invention concerne un procédé de préparation de particules de stiripentol ayant une granulométrie définie.
Le stiripentol (STP), encore désigné 4-diméthyl-1-[(3,4-méthylènedioxy)-phényl]-1-penten-3-ol, est le composé de formule :

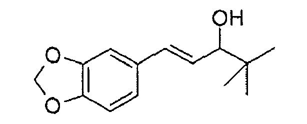

Le stiripentol appartient à la famille des alcools α-éthyléniques ayant une activité sur le système nerveux central. Il est susceptible de traiter les maladies du système nerveux central et est notamment utilisé comme médicament (dénommé Diacomit®) pour son effet antiépileptique, par exemple chez les patients souffrant d'épilepsie myoclonique sévère du nourrisson.
Diacomit® se présente sous forme de gélules contenant 250 mg ou 500 mg de stiripentol ou sous forme de granulés pour suspension buvable en sachet à 250 et 500 mg. La granulométrie du stiripentol contenu dans ces formulations est telle que le diamètre de 50 % (en nombre) des particules (ci-après désigné d₅₀) est inférieur ou égal à 100 µm, notamment compris entre 50 et 85 µm, et le diamètre de 90 % (en nombre) des particules (ci-après désigné d₉₀) est inférieur ou égal à 300 µm, notamment inférieur ou égal à 250 µm.
Ces particules peuvent être obtenues selon des procédés classiques de broyage et de tamisage pour obtenir la granulométrie définie. Cependant, le broyage pose des problèmes car, du fait de sa température de fusion relativement basse (Tf=75°C), le stiripentol a tendance à fondre au cours de la manipulation, ce qui conduit à un collage des particules entre elles et donc à des pertes de produit importantes.
Le brevet FR 2 253 504 décrit la cristallisation du stiripentol dans l'éthanol. Cependant, aucune indication n'est mentionnée quant à la granulométrie du produit obtenu. En outre, le stiripentol étant très soluble dans l'éthanol à température ambiante, ce procédé de cristallisation est difficilement transposable à l'échelle industrielle.

Il a maintenant été découvert un procédé permettant de préparer des particules de stiripentol ayant la granulométrie souhaitée, avec de bons rendements, et pouvant être réalisé à l'échelle industrielle. Plus précisément, il a été montré qu'en procédant à une recristallisation du stiripentol dans un solvant aromatique, il était possible d'obtenir directement la granulométrie recherchée, sans étapes de broyage et/ou de tamisage subséquentes. En outre, ce procédé permet d'obtenir un produit d'une bonne pureté.

Ainsi, selon un premier aspect, l'invention concerne un procédé de préparation de particules de stiripentol, comprenant :
i) la dissolution du stiripentol dans un solvant aromatique ;
ii) la cristallisation du stiripentol dans ledit solvant ;
iii) la récupération des particules de stiripentol obtenues.

De manière avantageuse, les particules de stiripentol obtenues possèdent la granulométrie recherchée, à savoir une granulométrie dans laquelle :
- d₅₀ est inférieur ou égal à 100 µm, notamment compris entre 50 et 85 µm, et
- d₉₀ est inférieur ou égal à 300 µm, notamment inférieur ou égal à 250 µm.

Cette granulométrie est mesurée par voie humide. A titre d'exemple, elle peut être mesurée au moyen d'un appareil Malvern Mastersizer 2000 SM équipé d'une cellule de mesure hydro 2000 SM, sur un échantillon de 100 mg de stiripentol mis en suspension dans 30 ml d'eau en présence d'un tensioactif.
De préférence, le solvant aromatique est choisi parmi le méta-, para-, orthoxylène, le dichlorobenzène, le toluène ou un mélange de ceux-ci, le toluène étant tout particulièrement préféré.
Si la concentration du stiripentol à l'étape i) ne constitue pas un facteur critique de la cristallisation, on préfère opérer en présence d'une concentration en stiripentol proche de la concentration de saturation dans le solvant considéré.
De façon privilégiée, à l'étape i), la concentration du stiripentol dans le solvant aromatique est comprise entre 1 et 2 kg/L (soit une concentration comprise entre 4 et 9 mol/L), de préférence d'environ 1 kg/L.

Typiquement, l'étape i) est réalisée en chauffant le stiripentol à une température comprise entre 70°C et 100°C jusqu'à complète dissolution, de préférence dans des conditions anhydres, notamment sous atmosphère inerte.
Afin d'éliminer d'éventuelles traces de coloration, des produits de type célite (ou "clarcel") ou charbon actif peuvent être ajoutés dans le mélange.

Selon une variante préférée, après dissolution du stiripentol et avant l'étape ii), les éventuelles traces d'eau présentes dans la solution sont éliminées par distillation azéotropique, en chauffant le mélange à reflux. En effet, en présence d'eau légèrement acide, le stiripentol peut conduire à des produits indésirables résultant notamment de la condensation du stiripentol sur lui-même.
Le processus de cristallisation du stiripentol à l'étape ii) peut être accéléré selon des techniques connues de l'homme du métier, à savoir le refroidissement de la solution, l'évaporation d'une partie du solvant, l'addition d'un anti-solvant ou l'ensemencement de la solution avec des cristaux de stiripentol. Le plus souvent, le mélange est maintenu sous agitation tout au long du processus de cristallisation, de manière à obtenir une suspension homogène et un renouvellement rapide de la liqueur-mère autour de chaque cristallite.

Selon un mode de réalisation préféré, la cristallisation du stiripentol est réalisée par refroidissement, typiquement avec une vitesse de refroidissement comprise entre -0,4 et -1,5°C/min, jusqu'à une température comprise généralement entre 0°C et -10 °C. Généralement, le mélange est maintenu à cette température pendant/sur une période d'environ 1 à 3 heures.
De préférence, la cristallisation du stiripentol est réalisée sous agitation. La vitesse d'agitation peut varier en fonction de la taille et de la géométrie du réacteur ainsi qu'en fonction du type de dispositif d'agitation. A noter cependant que le type de dispositif d'agitation est sans influence sur la granulométrie du stiripentol obtenu. Généralement, on opère à une vitesse comprise entre 75 et 125 rpm.
Enfin, les cristaux de stiripentol forment des particules qui peuvent être isolées à l'étape iii) selon des méthodes classiques telles que la filtration ou la centrifugation.

Selon une variante particulièrement préférée, le procédé selon l'invention comprend de plus une deuxième recristallisation, de préférence dans le même solvant aromatique que la première recristallisation. Plus précisément, le procédé comprend en outre une étape iv), subséquente à l'étape iii), de répétition des étapes i) à iii) à partir des particules de stiripentol récupérées à l'étape iii).
Avantageusement, cette double recristallisation permet d'améliorer la reproductibilité du procédé, la pureté du produit et d'obtenir une poudre homogène et incolore. En outre, lorsqu'elle est, selon un mode de réalisation privilégié, réalisée dans le toluène, le taux de toluène résiduel est inférieur à 500 ppm, soit très nettement inférieur à la norme ICH Q3C (International Conference of Harmonization) de 890 ppm.
De préférence, les particules de stiripentol récupérées, notamment après la deuxième recristallisation, sont lavées avec ledit solvant aromatique, puis séchées sous vide à une température suffisante pour éliminer les traces de solvant résiduel.

### EXEMPLES

### Exemple 1 : Préparation du (±)-(E)-4,4-dimethyl-1-[3,4-methylenedioxy)phényl]-1-penten-3-ol ou stiripentol

Le stiripentol est préparé à partir de la (méthylènedioxy-3,4-phényl)-1-diméthyl-4,4-penten-1-on-3 (n° code D305) préparé selon le brevet FR 2 173 691.
Dans un réacteur, sont ajoutés quatre volumes de méthanol par masse de cétone D305. On ajoute ensuite une solution aqueuse de borohydrure de potassium préparée à partir de borohydrure de potassium (0,12 kg par kg de cétone), d'eau (0,845 L par kg de cétone) et d'hydroxyde de sodium 2N (0,00235 L par kg de cétone). Le mélange est maintenu à une température de 20°-25°C, sous agitation et sous azote.
Le mélange est agité pendant une nuit à température ambiante. Le mélange réactionnel est ensuite dilué avec 5 L d'eau par kg de cétone de départ. Le pH est ensuite ajusté à une valeur comprise entre 8,5 et 9 avec de l'acide chlorhydrique. Le produit formé est ensuite séché par centrifugation et lavé de façon abondante. Le produit est ensuite séché dans un four ventilé à 60°C pendant 24 heures.

### Première recristallisation :

Le produit obtenu est ensuite dilué dans le toluène à raison de 1 L de toluène par kg de produit sec. Le mélange est ensuite porté à 80°-90°C jusqu'à dissolution complète du stiripentol. On ajoute ensuite 2 kg de clarcel et 3 kg de charbon actif. Le mélange est ensuite chauffé à 110°C à reflux, et on élimine par distillation azéotropique toute trace d'eau résiduelle dans le produit. Le mélange est filtré puis refroidi à une température de - 5°C pendant une heure. Le mélange est ensuite séché par centrifugation.

### Deuxième recristallisation :

Le stiripentol obtenu à l'issue de cette première recristallisation est ensuite dissous dans une quantité de toluène équivalente à celle mise en oeuvre dans la première purification. Le mélange est ensuite chauffé à 110°C au reflux et filtré de manière à éliminer toute impureté solide. Le mélange est ensuite refroidi à - 5°C aussi rapidement que possible, sous forte agitation (100 rpm) et maintenu à cette température pendant une heure. Le produit est ensuite séché par centrifugation et lavé avec environ 50 L de toluène.

### Séchage du produit :

Le produit est ensuite séché sous vide à 40°C pendant 24 heures (jusqu'à une masse constante). Afin d'homogénéiser les lots et d'éviter la présence d'éventuels agrégats pouvant être formés pendant le séchage, le produit peut être broyé avec une grille de taille de mesh de 1,5 mm. Le produit peut être à nouveau séché sous vide dans un four à 50°C pendant 24 heures. Le produit peut être criblé à travers une grille de taille de mesh de 630 µm et mélangé.
Rendement moyen : 85 %
Point de fusion : 75°C.
Diamètre moyen (d₅₀) : de 50 à 85 µm.
Diamètre de 90% des particules (d₉₀) ≤ 250 µm.

## Revendications

1. Procédé de préparation de particules de stiripentol **possédant une granulométrie dans laquelle :**
- **le diamètre d₅₀ est compris entre 50 et 85 µm, et**
- **le diamètre d₉₀ est inférieur ou égal à 300 µm**,
ledit procédé comprenant :
i) la dissolution du stiripentol dans un solvant aromatique ;
ii) la cristallisation du stiripentol dans ledit solvant ;
iii) la récupération des particules de stiripentol obtenues.

2. Procédé selon la revendication 1, dans lequel le solvant aromatique est choisi parmi le méta-, para-, orthoxylène, le dichlorobenzène, le toluène ou un mélange de ceux-ci.

3. Procédé selon la revendication 2, dans lequel le solvant aromatique est le toluène.

4. Procédé selon l'une quelconque des revendications 1 à 3, dans lequel, à l'étape i), la concentration de stiripentol dans le solvant aromatique est comprise entre 1 et 2 kg/L.

5. Procédé selon l'une quelconque des revendications 1 à 4, dans lequel la dissolution du stiripentol dans le solvant aromatique est réalisée en chauffant le mélange à une température comprise entre 70°C et 100°C.

6. Procédé selon l'une quelconque des revendications 1 à 5, dans lequel, après dissolution du stiripentol et avant l'étape ii), les éventuelles traces d'eau présentes dans la solution sont éliminées par distillation azéotropique.

7. Procédé selon l'une quelconque des revendications 1 à 6, dans lequel la cristallisation du stiripentol est réalisée par refroidissement.

8. Procédé selon la revendication 7, dans lequel la vitesse de refroidissement est comprise entre -0,4 et -1,5°C/min.

9. Procédé selon l'une quelconque des revendications 1 à 8, dans lequel la cristallisation du stiripentol est réalisée sous agitation.

10. Procédé selon la revendication 9, dans lequel la vitesse d'agitation est comprise entre 75 et 125 rpm.

11. Procédé selon l'une quelconque des revendications 1 à 10, comprenant de plus une répétition des étapes i) à iii) à partir des particules de stiripentol obtenues à l'étape iii).

12. Procédé selon l'une quelconque des revendications 1 à 10, dans lequel les particules de stiripentol récupérées sont lavées avec ledit solvant aromatique.

## Claims

1. Method of preparing stiripentol particles of a specific particle size, in which:
- the diameter d₅₀ is between 50 and 85 µm, and
- the diameter d₉₀ is less than or equal to 300 µm,
said method comprising:
i) dissolving the stiripentol in an aromatic solvent;
ii) crystallising the stiripentol in said solvent;
iii) recovering the stiripentol particles obtained.

2. Method as claimed in claim 1, in which the aromatic solvent is selected from meta-xylene, para-xylene, ortho-xylene, dichlorobenzene, toluene or a mixture thereof.

3. Method as claimed in claim 2, in which the aromatic solvent is toluene.

4. Method as claimed in any one of claims 1 to 3, in which the concentration of stiripentol in the aromatic solvent in step i) is between 1 and 2 kg/L.

5. Method as claimed in any one of claims 1 to 4, in which the stiripentol is dissolved in the aromatic solvent by heating the mixture to a temperature of between 70°C and 100°C.

6. Method as claimed in any one of claims 1 to 5, in which any traces of water present in the solution after dissolving the stiripentol are removed by azeotropic distillation before step ii).

7. Method as claimed in any one of claims 1 to 6, in which the stiripentol is crystallised by cooling.

8. Method as claimed in claim 7, in which the cooling rate is between -0.4 and -1.5°C/min.

9. Method as claimed in any one of claims 1 to 8, in which the stiripentol is crystallised under agitation.

10. Method as claimed in claim 9, in which the agitation speed is between 75 and 125 rpm.

11. Method as claimed in any one of claims 1 to 10, further comprising a repetition of steps i) to iii) using the stiripentol particles obtained from step iii).

12. Method as claimed in any one of claims 1 to 10, in which the recovered stiripentol particles are washed with said aromatic solvent.

## Patentansprüche

1. Verfahren zur Herstellung von Stiripentol-Teilchen, die eine Granulometrie besitzen, bei der:
- der Durchmesser d₅₀ zwischen 50 und 85 µm liegt und
- der Durchmesser d₉₀ unter oder gleich 300 µm ist,
wobei das Verfahren umfasst:
i) Auflösung des Stiripentol in einem aromatischen Lösungsmittel;
ii) Kristallisation des Stiripentol in dem Lösungsmittel;
iii) Gewinnung der erhaltenen Stiripentol-Teilchen.

2. Verfahren gemäß Anspruch 1, in dem das aromatische Lösungsmittel unter meta-, para-, ortho-Xylol, Dichlorbenzol, Toluol und einem Gemisch aus zwei der Genannten ausgewählt wird.

3. Verfahren gemäß Anspruch 2, wobei das aromatische Lösungsmittel Toluol ist.

4. Verfahren gemäß einem der Ansprüche 1 bis 3, wobei in Stufe i) die Konzentration an Stiripentol in dem aromatischen Lösungsmittel zwischen 1 und 2 kg/L ist.

5. Verfahren gemäß einem der Ansprüche 1 bis 4, wobei die Auflösung des Stiripentol in dem aromatischen Lösungsmittel unter Erwärmen des Gemisches auf eine Temperatur zwischen 70°C und 100°C durchgeführt wird.

6. Verfahren gemäß einem der Ansprüche 1 bis 5, wobei nach Auflösung des Stiripentols und vor Stufe ii) mögliche Spuren an Wasser, die in der Lösung vorliegen, durch azeotrope Destillation entfernt werden.

7. Verfahren gemäß einem der Ansprüche 1 bis 6, wobei die Kristallisation des Stiripentol durch Kühlung durchgeführt wird.

8. Verfahren gemäß Anspruch 7, wobei die Kühlgeschwindigkeit zwischen -0,4 und -1,5°C/min ist.

9. Verfahren gemäß einem der Ansprüche 1 bis 8, wobei die Kristallisation des Stiripentol unter Rühren durchgeführt wird.

10. Verfahren gemäß Anspruch 9, wobei die Rührgeschwindigkeit zwischen 75 und 125 upm liegt.

11. Verfahren gemäß einem der Ansprüche 1 bis 10, das außerdem eine Wiederholung der Stufen i) bis iii) ausgehend von Stiripentol-Teilchen, die in Stufe iii) erhalten wurden, umfasst.

12. Verfahren gemäß einem der Ansprüche 1 bis 10, wobei die gewonnenen Stiripentol-Teilchen mit dem aromatischen Lösungsmittel gewaschen werden.
